# EUROPEAN PATENT SPECIFICATION

(11) **EP 4 312 846 B1**
(45) Date of publication and mention of the grant of the patent: **28.10.2026**
(21) Application number: 22715638.7
(22) Date of filing: 31.03.2022
(51) Int. Cl.: A61B 18/14, A61B 5/00, A61B 5/06, A61B 18/00

(54) **IMPEDANCE GUIDED POSITIONING OF THE CATHETER TO REDUCE CONTRAST EXPOSURE**
IMPEDANZGEFÜHRTE POSITIONIERUNG DES KATHETERS ZUR REDUZIERUNG VON KONTRASTEXPOSITION
POSITIONNEMENT GUIDÉ PAR IMPÉDANCE DU CATHÉTER POUR RÉDUIRE L'EXPOSITION AU CONTRASTE

(30) Priority: 31.03.2021 US 202163168810 P
(43) Date of publication of application: 07.02.2024
(73) Proprietor: Medtronic Ireland Manufacturing Unlimited Company, Dublin 2, D02 T380 (IE)
(72) Inventor: MAUCH, Kevin, Santa Rosa, California 95403 (US)
(74) Representative: Zimmermann & Partner Patentanwälte mbB
(86) International application number: PCT/EP2022/058673
(87) International publication number: WO 2022/207849

(56) References cited:
- EP-A1- 3 571 989
- US-A1- 2017 007 157
- US-A1- 2018 360 342

## Description

### BACKGROUND

### 1. Field

This specification relates to a system, a device, a method and/or an apparatus for determining the location of an intravascularly placed catheter and one or more energy delivery elements.

### 2. Description of the Related Art

Renal denervation is a minimally invasive procedure to treat resistant hypertension. During renal denervation, a nurse, doctor, technician or other hospital staff (or "clinician") uses stimuli or energy, such as radiofrequency, ultrasound, cooling or other energy, to perform ablation within the renal arteries. This reduces activity of the nerves surrounding the vessel, which has been shown to result in a decrease in blood pressure and other benefits. The clinician uses the renal denervation device to deliver the stimuli or energy to the treatment site, e.g., through one or more energy delivery elements of the renal denervation device.

The renal denervation device may deliver neuromodulation energy, such as radiofrequency (RF) energy, through the one or more energy delivery elements to the treatment site, which heats the wall of the vessel, and as a consequence, warms the energy delivery element in contact with the wall of the vessel. Before the delivery of neuromodulation energy, however, the clinician must locate the renal denervation device within the blood vessel to the treatment site, such as within the renal artery, so that the one or more energy delivery elements are positioned at or near the treatment site. In order to locate the renal denervation device, the clinician may introduce or inject contrast dye into the areas surrounding the blood vessel to enable the clinician to visualize the area of interest via fluoroscopy. Moreover, as the clinician performs the renal denervation, the clinician may need to monitor the location of the renal denervation device so that the treatment may be monitored, and as such, the clinician may need to reintroduce additional contrast dye to perform fluoroscopy to visualize the area of interest. The introduction of contrast dye into the blood vessel and/or other organs may adversely impact the blood vessel and/or other organs, such as the kidney. US 2017/007157 A1 relates to an electrical-signal-based electrode-tissue contact detection. US 2018/360342 A1 relates to renal ablation and a visualization system and method with a composite anatomical display image. EP 3 571 989 A1 relates to scaling impedance location measurements of a balloon catheter.

Thus, the reliance of the clinician on fluoroscopy, which may use the introduction of a contrast dye to visualize the location of the renal denervation device, may adversely affect the blood vessel and/or organs, such as cause contrast induced nephropathy. Accordingly, there is a need for a system, apparatus, device and/or method to detect the location of the renal denervation device and its components safely and reliably without the use of contrast dye.

### SUMMARY

The invention is defined by the claims. In the following any method of renal denervation is not claimed and is shown for illustrative purposes only. In general, one aspect of the subject matter described in this specification is embodied in a system for renal denervation. The system includes a guide catheter. The guide catheter has a lumen and a guide catheter energy delivery element positioned at a distal portion of the guide catheter. The distal portion of the guide catheter is configured to be intravascularly positioned within a main vessel. The system include an inner catheter. The inner catheter is positioned within the lumen of the guide catheter and has multiple energy delivery elements. The multiple energy delivery elements include a first energy delivery element and a second energy delivery element. The inner catheter has a distal portion that is configured to be positioned near a treatment site within a renal blood vessel and transform from a delivery configuration to a deployed configuration. The system includes a controller. The controller is coupled to the renal denervation device. The controller is configured to measure a first impedance between the first energy delivery element and the guide catheter energy delivery element. The controller is configured to provide a location of the inner catheter within the renal blood vessel based on the first impedance.

These and other embodiments may optionally include one or more of the following features. When the inner catheter is in the delivery configuration, the inner catheter may be substantially within the lumen of the guide catheter and the energy delivery element may be aligned with the guide catheter energy delivery element. The controller may be configured to calibrate the impedance between the energy delivery element and the guide catheter energy delivery element when the inner catheter is in the delivery configuration. When the inner catheter is in the deployed configuration, the inner catheter may be extended away from the guide catheter and a distal portion of the inner catheter may extend into a helical or spiral configuration to place the energy delivery element at or near the treatment site.

The controller may be configured to measure the first impedance between the first energy delivery element and the guide catheter when the inner catheter is in the delivery configuration. The controller may be configured to measure a second impedance between the first energy delivery element and the guide catheter energy delivery element when the inner catheter is in the deployed configuration. The controller may be configured to determine a difference between the second impedance and the first impedance. The controller may be configured to determine the location of the inner catheter based on the difference.

The controller may be configured to measure a third impedance between the second energy delivery element and the guide catheter energy delivery element when the inner catheter is in the deployed configuration. The controller may be configured to determine a second difference between the third impedance and the first impedance. The controller may be configured to determine the location of the inner catheter including a location of the first energy delivery element and a location of the second delivery element based on the first difference and the second difference.

The system may include a display. The display may be configured to render an image of the renal blood vessel, the guide catheter and the inner catheter including the first energy delivery element and the second energy delivery element within the renal blood vessel. The controller may be configured to generate an image of the renal blood vessel, the guide catheter and the inner catheter within the renal blood vessel. The controller may be configured to cause the image to be rendered on the display.

In another aspect, the subject matter is embodied in a therapeutic assembly. The therapeutic assembly includes a guide catheter. The guide catheter has a lumen and a guide catheter energy delivery element positioned at a distal portion of the guide catheter. The distal portion is configured to be intravascularly positioned within a renal blood vessel. The renal denervation device includes an inner catheter. The inner catheter is positioned within the lumen of the guide catheter and has an energy delivery element. The inner catheter is configured to be positioned near a treatment site within the renal blood vessel. The therapeutic assembly includes a display. The display is configured to render images. The therapeutic assembly includes a controller. The controller is coupled to the guide catheter, the inner catheter and the display. The controller is configured to measure a first impedance between the energy delivery element and the guide catheter energy delivery element. The controller is configured to determine a location of the inner catheter based on the first impedance. The controller is configured to render, on the display, an image of the guide catheter and the inner catheter based on the location of the inner catheter within renal blood vessel.

In another aspect, the subject matter is embodied in a method of renal denervation. The method includes intravascularly positioning a guide catheter having a lumen and a guide catheter energy delivery element positioned on a distal portion of the guide catheter. The method includes obtaining, by a processor, an initial image of a renal blood vessel and the guide catheter. The method includes positioning an inner catheter having a first energy delivery element and a second energy delivery element at or near a treatment site within the renal blood vessel. The method includes measuring, by a processor, a first impedance between the first energy delivery element and the guide catheter energy delivery element to determine a location of the inner catheter. The method includes generating, by the processor, an overlay of an image of the inner catheter on the initial image of the renal blood vessel and the guide catheter based on the location of the inner catheter.

### BRIEF DESCRIPTION OF THE DRAWINGS

Other systems, methods, features, and advantages of the present invention will be or will become apparent to one of ordinary skill in the art upon examination of the following figures and detailed description. It is intended that all such additional systems, methods, features, and advantages be included within this description, be within the scope of the present invention, and be protected by the accompanying claims. Component parts shown in the drawings are not necessarily to scale and may be exaggerated to better illustrate the important features of the present invention. In the drawings, like reference numerals designate like parts throughout the different views.
FIG. 1 shows an example conceptual illustration of the therapeutic assembly according to an aspect of the invention.
FIG. 2A shows an example renal denervation device of the therapeutic assembly of FIG. 1 in a low-profile delivery configuration according to an aspect of the invention.
FIG. 2B shows an example renal denervation device of the therapeutic assembly of FIG. 1 in an expanded deployed configuration according to an aspect of the invention.
FIG. 3A shows an example guide catheter and renal denervation device of the therapeutic assembly of FIG. 1 being deployed within a blood vessel according to an aspect of the invention.
FIG. 3B shows another example of the guide catheter and renal denervation device of the therapeutic assembly of FIG. 1 being deployed within a blood vessel according to an aspect of the invention.
FIG. 3C shows an example renal denervation device of the therapeutic assembly of FIG. 1 in the expanded deployed configuration within a blood vessel according to an aspect of the invention.
FIG. 4 shows a front view of the distal portion of the renal denervation device of the therapeutic assembly of FIG. 1 that looks down a longitudinal axis of the blood vessel according to an aspect of the invention.
FIG. 5 is a block diagram of an example generator of the therapeutic assembly of FIG. 1 according to an aspect of the invention.
FIG. 6 is a flow diagram of a process for providing an image of the renal denervation device within the blood vessel using the therapeutic assembly of FIG. 1 according to a non-claimed method.
FIG. 7 is a flow diagram of a process for determining the locations of one or more energy delivery elements within the blood vessel using the therapeutic assembly of FIG. 1
FIG. 8 shows the trilateration or triangulation of the locations of the one or more energy delivery elements within the blood vessel using the therapeutic assembly of FIG. 1
FIG. 9 is a flow diagram of a process for tracking and plotting the movement of the renal denervation device within the blood vessel using the therapeutic assembly of FIG. 1 according to a non-claimed method.
FIG. 10A shows an example fluoro image of the renal artery according to an aspect of the invention.
FIG. 10B shows an example image of the renal artery that would appear on the screen of the user interface of the therapeutic assembly of FIG. 1 to track the location of the guide catheter and the inner catheter according to an aspect of the invention.
FIG. 10C shows an example image that would appear on the screen of the user interface of the therapeutic assembly of FIG. 1 as the inner catheter is tracked within the renal artery according to an aspect of the invention.

### DETAILED DESCRIPTION

Disclosed herein are systems, devices, methods and/or apparatuses for a therapeutic assembly including a renal denervation device that delivers neuromodulation energy from one or more energy delivery elements and measures, detects or otherwise obtains parameters, such as impedance, to facilitate determination of the location of the renal denervation device and/or the one or more energy delivery elements within a blood vessel, such as within the renal artery. Typically, a clinician may introduce a contrast dye into the area of interest, such as within the blood vessel or other organ, and then the clinician may perform fluoroscopy to render an image of the renal denervation device within the blood vessel. The introduction of contrast dye to perform the fluoroscopy, however, may cause an increased risk of adverse impacts to the blood vessel and/or the organ, such as an increased risk of contrast induced nephropathy in the kidney. And thus, by determining the location of the renal denervation device and/or the one or more energy delivery elements within the blood vessel without the use contrast dye, the therapeutic assembly reduces or minimizes contrast usage and reduces the risk of adverse impacts, such as to the kidney

Other benefits and advantages of the therapeutic assembly including built-in sensors that measure, detect or otherwise obtain parameters related to the extension of the inner catheter away from the guide catheter within the blood vessel. Since the sensors are built-in or included within the therapeutic assembly a separate additional device to detect, measure or obtain the parameters is unnecessary, which reduces complexity and costs of the renal denervation (RDN) procedure. Additionally, the therapeutic assembly may record, store or otherwise track the location of the renal denervation device over a period of time and/or before, during or after multiple treatments. By tracking the location of the renal denervation device over the period of time, the therapeutic assembly may plot the treatment locations on the fluoroscopy image as well as keep track of when and where the treatments occurred. This provides the clinician a historical reference of the parameters of the treatments including the location, timing, amount, type and/or other parameters of the treatments.

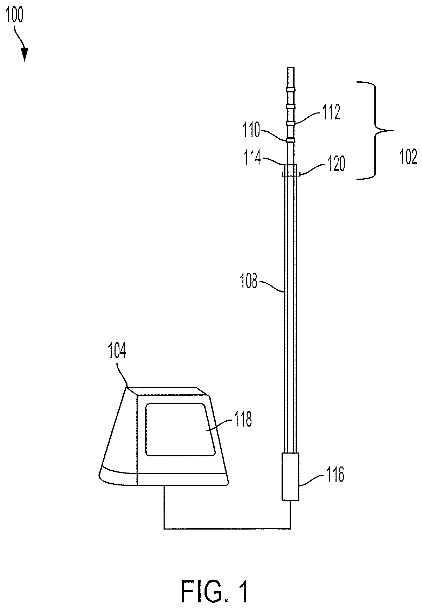

FIG. 1 shows the therapeutic assembly 100. The therapeutic assembly 100 performs renal denervation within the renal artery of a human patient. Renal denervation is a minimally invasive procedure to treat hypertension. The therapeutic assembly 100 includes a renal denervation device 102, a guide catheter 108 and/or a generator 104. The renal denervation device 102 may include any device that delivers energy or stimulus to a target nerve within a wall of a blood vessel, such as the renal nerve of the renal artery. The energy or stimulus may include, for example, at least one of a radio frequency stimulus, a thermal stimulus, a cryogenic stimulus, a microwave stimulus, an ultrasonic stimulus or other form of energy or stimulus.

The guide catheter 108 may have a guide catheter energy delivery element 120. The guide catheter 108 may be coupled to or integral with the renal denervation device 102. The renal denervation device 102 may have an inner catheter or shaft (hereinafter, referred to as "inner catheter") 114, one or more energy delivery elements 110 and/or one or more sensors 112. The one or more sensors 112 may be integrated with the one or more energy delivery elements 110 and/or be the same component as the one or more energy delivery elements 110. The guide catheter energy delivery element 120 and/or the one or more energy delivery elements 110 may be an electrode or other neuromodulation element that delivers ablative, therapeutic and/or neuromodulation energy.

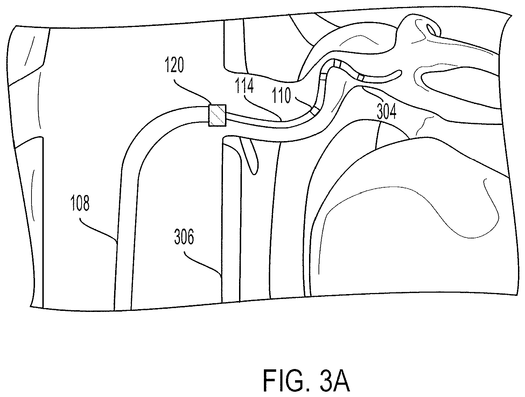

The therapeutic assembly 100 may have a handle 116. The handle 116 may be used to guide and/or advance a distal portion of the guide catheter 108 through the blood vessels of the patient, such as a human patient, to a target location of a blood vessel and remotely manipulate the distal portion of the guide catheter 108, as shown in FIG. 3A for example. The guide catheter 108 may be advanced into the vasculature and up to the renal artery independent of the inner catheter 114. The guide catheter 108 may be temporarily anchored near the ostium of the renal artery. The guide catheter 108 may be over a meter in length and have a lumen that encloses or surrounds an inner catheter 114.

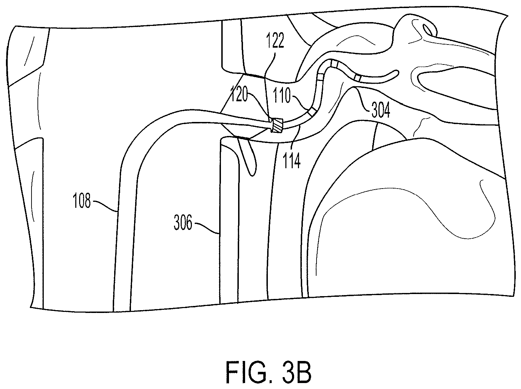

The guide catheter 108 may have an expandable element 122, as shown in FIG. 3B for example. The expandable element 122 may be a balloon or an expandable wire element. The expandable element stabilizes the guide catheter 108 within the blood vessel 304 so that the guide catheter 108 does not move relative to the inner catheter 114 after the initial calibration. This prevents the guide catheter 108 from being retracted, repositioned or otherwise moved relative to the inner catheter 114 so that the measurements of the impedance are not compromised. The expandable element 122 may be designed to partially or fully occlude the blood flow within the renal artery and anchor the guide catheter 108 within the renal artery to prevent lateral translation of the guide catheter 108 during the neuromodulation procedure. In some implementations, the expandable element 122 is made of nickel titanium and may be positioned at a distal end of the guide catheter 108 to anchor the guide catheter 108 within the renal artery. In some implementations, the guide catheter energy delivery element 120 is distal to the expandable element 122. In other implementations, the guide catheter energy delivery element 120 may be positioned on the expandable element 122 such that the guide catheter energy delivery element 120 is pressed against the blood vessel 304.

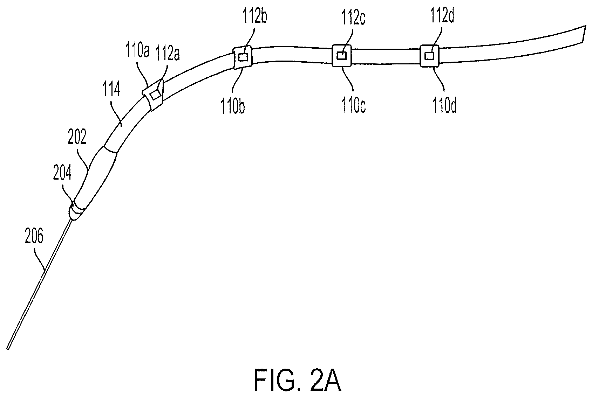

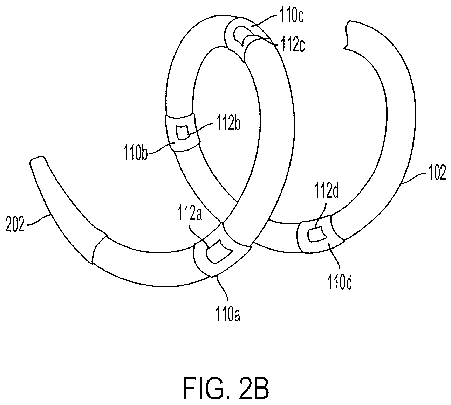

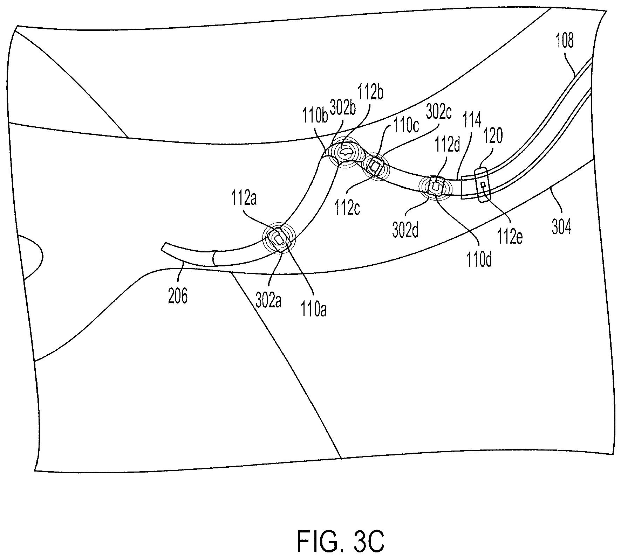

The inner catheter 114 may then be inserted into a proximal end of the guide catheter 108 and advanced up to and/or beyond a distal end of the guide catheter 108 into the renal artery towards the treatment site. The inner catheter 114 may be in a delivery configuration, such that the inner catheter 114 is positioned partially or completely within a lumen of the guide catheter 108, when the guide catheter 108 is being intravascularly delivered into the patient. Upon delivery to a target location within, near and/or along the blood vessel, the inner catheter 114 may be extended or moved away from the distal portion of the guide catheter 108 and deployed into an expanded deployed configuration, such as a generally helical or spiral configuration or other suitable configuration, where the one or more energy delivery elements 110, such as one or more electrodes, may contact the blood vessel 304, as shown in FIG. 3C for example. In the expanded deployed state, the renal denervation device 102 may deliver energy at a treatment site and provide therapeutically-effective electrically and/or thermally induced denervation to a nerve within the wall of the blood vessel 304. The inner catheter 114 may be deployed in a generally helical or spiral configuration that rotates clockwise or counter-clockwise relative to a longitudinal central axis. FIGS. 2A-2B show the deployment of the renal denervation device 102. In particular, FIG. 2A shows the inner catheter 114 in the low-profile configuration, and FIG. 2B shows the inner catheter 114 in the expanded deployed configuration.

The inner catheter 114 may have a distal tip 202. The distal tip 202 points into the lumen of the blood vessel. The distal tip 202 may have a high density radiopaque marker band 204. The high density radiopaque marker band 204 allows a clinician to identify the distal tip 202 of the inner catheter 114 under fluoroscopy because the tip of the catheter may not be in optimal contact with the vessel wall compared to the more proximal energy delivery elements along the spiral portion of the device. The distal portion of the inner catheter 114 may be approximately 4 cm - 5 cm in length, and the distal tip 202 may be approximately 1 cm - 2 cm in length. In some implementations, there may be an electrical connection or wire between the radiopaque marker band 204 and a proximal end of the inner catheter 114 such that the radiopaque marker band 204 at the distal tip of the inner catheter 114 may function as an energy delivery element.

The inner catheter 114 may utilize a guidewire 206 within the lumen of the inner catheter 114. The distal tip 202 allows the guidewire 206 to extend out and away from the distal tip 202 when the inner catheter 114 is in the low-profile delivery configuration and to be advanced through the blood vessels to the target location of the blood vessel. When the guidewire 206 is retracted within the distal tip 202 and into the inner catheter 114, the inner catheter 114 changes shape from the low-profile delivery configuration, such as the substantially straight configuration, as shown in FIG. 2A for example, to the expanded deployed configuration, such as a generally helical or spiral configuration, as shown in FIG. 2B for example.

The renal denervation device 102 has one or more energy delivery elements 110. The guide catheter 108 may have one or more guide catheter energy delivery elements 120. The one or more energy delivery elements 110 may include an electrode, such as a radiofrequency (RF) electrode, a radiofrequency (RF) probe, a thermal probe, a cryogenic probe, a microwave probe, an ultrasonic probe, an optical source or a chemical injector. The one or more energy delivery elements 110 may be positioned on the distal portion of the inner catheter 114 and the guide catheter energy delivery element 120 may be positioned on the distal portion of the guide catheter 108. The one or more energy delivery elements 110 may include multiple energy delivery elements 110, such as the energy delivery elements 110a-d, as shown in FIGS. 2A, 2B and 3 for example, or any other N number of energy delivery elements 110. The energy delivery elements 110a-d may be arranged approximately 90 degrees apart relative to a longitudinal axis that runs through the center of the guide catheter 108 when in the spiral configuration. The energy delivery elements 110 may be spaced any suitable distance from each other, and the spacing may vary based on the application of the therapeutic assembly 100 and its intended use.

When there are multiple energy delivery elements 110, each energy delivery element 110 may deliver power independently, either simultaneously, selectively, and/or sequentially, to a treatment site. The multiple energy delivery elements 110 may deliver power among any desired combination of the one or more energy delivery elements 110. Similarly, the guide catheter energy delivery element 120 may deliver power independently of the one or more energy delivery elements 110, either simultaneously, selectively, and/or sequentially, to a treatment site.

The guide catheter 108 may be introduced and advanced through a main blood vessel 306, such as the aorta, and as the guide catheter 108 is advanced through the main blood vessel 306 the guide catheter 108 may flex through the tortuous path of the vasculature. The guide catheter 108 may be positioned proximate to or through a blood vessel 304, such as the renal artery, that branched from the main blood vessel 306. The guide catheter energy delivery element 120 may be positioned at or near an entrance of the blood vessel 304 and allow the inner catheter 114 to be extended away from and expand into the deployed configuration so that the one or more energy delivery elements 110 may be positioned to contact the blood vessel 304 in the expanded deployed configuration at different intervals and/or locations along the wall of the blood vessel 304.

For example, the guide catheter energy delivery element 120 may be within the vasculature of the blood vessel 304, the first energy delivery element 110a may contact the wall of the blood vessel 304 at a first location 302a, the second energy delivery element 110b may contact the wall of the blood vessel 304 at a second location 302b, the third energy delivery element 110c may contact the wall of the blood vessel 304 at a third location 302c and the fourth energy delivery element 110d may contact the wall of the blood vessel 304 at a fourth location 302d. The renal denervation device 102 may deliver energy through the one or more energy delivery elements 110 and/or the guide catheter energy element 120 at or near the treatment sites and provide therapeutically-effective electrically- and/or thermally- induced denervation.

The renal denervation device 102 may include one or more sensors 112. The one or more sensors 112 may measure one or more parameters at or near the treatment site. The one or more parameters may include a temperature, an impedance, a pressure, an optical, a flow or an amount of chemical. For example, the one or more sensors 112 may be an impedance sensor positioned along the inner catheter 114 and/or along the guide catheter 108. The impedance sensor may measure the impedance at or near a location of the wall of the blood vessel and/or the impedance between or among the one or more energy delivery elements 110 and/or the guide catheter energy delivery element 120.

Each of the one or more sensors 112a-e may be coupled or integrated with a corresponding one of the one or more energy delivery elements 110 and/or the guide catheter energy delivery element 120. For example, the sensor 112a may be integrated with the first energy delivery element 110a, the sensor 112b may be integrated with the second energy delivery element 110b, the sensor 112c may be integrated with the third energy delivery element 110c, the sensor 112d may be integrated with the fourth energy delivery element 110d, and the sensor 112e may be integrated with the guide catheter energy delivery element 120, as shown in FIG. 3B for example.

The one or more sensors 112 may be proximate to or within a corresponding energy delivery element 110 and/or the guide catheter energy delivery element 120. The one or more sensors 112 may be sufficiently close or proximate to the corresponding energy delivery element 110 and/or the guide catheter energy delivery element 120 so that the one or more sensors 112 experience any change in surface area of the one or more sensors 112 and/or the corresponding energy delivery element 110 and/or the guide catheter energy delivery element 120 in contact with the wall of the blood vessel 304 and/or in contact with the blood that flows through the lumen of the blood vessel 304.

In some implementations, the one or more sensors 112 may be integrated with the one or more energy delivery elements 110 and/or the guide catheter energy delivery element 120. For example, the energy delivery element 110 may be an electrode, which has two wires. One wire may be made from copper and the other may be made from a copper-nickel alloy. The wires may both transmit the signal from the sensor 112 and also convey the energy to the energy delivery element 110. Similarly, the guide catheter energy delivery element 120 may have electrical wiring that runs to the generator 104 via the handle 116.

The one or more sensors 112 may measure or calculate an impedance at or near the treatment site and/or between or among the one or more energy delivery elements 110 and/or the guide catheter energy delivery element 120. The impedance may be measured from one energy delivery element 110 to another energy delivery element 110 or the guide catheter energy delivery element 120 and/or from one energy delivery element 110 or the guide catheter energy delivery element 120 to another sensor, such as a grounding patch (commonly referred to as an "indifferent electrode") attached to the outside skin of the patient.

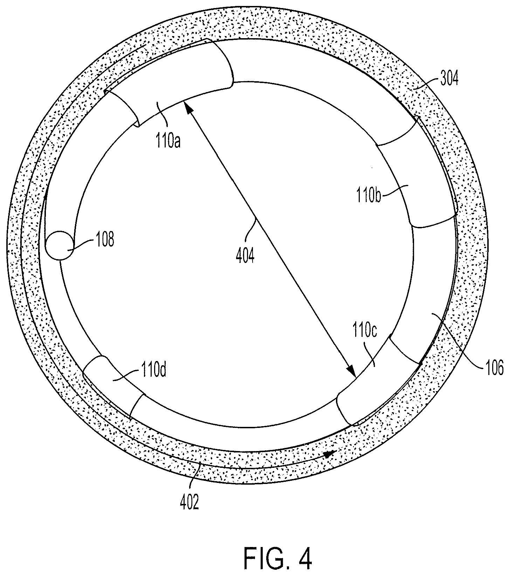

FIG. 4 shows a front view of the distal portion of the renal denervation device 102 that looks down a longitudinal axis of the blood vessel 304. For example, one of the energy delivery elements 110a-d may generate a signal and propagate the signal between the one or more energy delivery elements 110a-d through a path in the wall of the blood vessel 304, as indicated by the arrow 402, or through the blood or other medium in the lumen of the blood vessel 304, as indicated by the arrow 404. Moreover, the signal can be detected at multiple ones of the energy delivery elements 110, at the guide catheter energy delivery element 120 and/or at another sensor, such as a grounding patch. The degree of attenuation of the signal, the difference in the propagation time of the signal and/or other parameters of the signal may be correlated to the amount of blood between the energy delivery elements 110, the amount of surface area of the energy delivery element 110 that is in contact with the wall of the blood vessel 304, the distance between the energy delivery elements 110, 120, and/or the conduction path of the signal that travelled through the blood vessel. These measurements may indicate a position of the energy delivery element 110 relative to the other energy delivery elements 110 and/or the guide catheter energy delivery element 120. Changes in those parameters may also correlate and/or correspond to changes in the dimension of the blood vessel 304 and/or successful, partially successful or unsuccessful ablation of the treatment site. Changes in the vessel diameter may be directly correlated or proportionate to changes in blood pressure, since the artery is compliant.

In some implementations, the therapeutic assembly 100 may use the "four electrode" technique where the proximal and distal energy delivery elements drive a constant current field and the voltage is measured between the two electrodes to determine the impedance of the vessel segment bounded by the two energy elements. For example, the one or more sensors 112 may measure the impedance from a first energy delivery element to a second energy delivery element, such as from an ablation electrode to a dispersive electrode or from a guide catheter energy delivery element 120 on the guide catheter 108 to an energy delivery element 110 on the inner catheter 114.

The therapeutic assembly includes a generator 104. The generator 104 may be a radio frequency generator or other generator that delivers a denervation stimulus or energy through the one or more energy delivery elements 110 and/or the guide catheter energy delivery element 120 to the wall of the blood vessel at or near the treatment location. The denervation stimulus may include a non-electric stimulus, for example, a chemical agent, optical stimulus, a thermal stimulus, a cooling stimulus, a microwave stimulus or other form of stimuli. The generator 104 may have one or more cables, one or more electrical leads and/or one or more wires that are electrically conductive and run through the guide catheter 108 and/or the inner catheter 114 within a lumen and are electrically coupled with the one or more energy delivery elements 110 and/or the guide catheter energy delivery element 120.

In some implementations, the generator 104 may have one or more separate leads and/or wires that electrically couple with a corresponding energy delivery element 110 of the one or more energy delivery elements 110 and/or the guide catheter energy delivery element 120 so that each energy delivery element may operate independently of the others. For example, the generator 104 may have multiple separate channels, such as four radio frequency (RF) channels to deliver RF energy independently to the one or more energy delivery elements 110a-d and/or the guide catheter energy delivery element 120 and control and monitor each energy delivery element 110a-d and/or the guide catheter energy delivery element 120 independently. The generator 104 may generate energy that ultimately is transmitted through the electrical lead to the one or more energy delivery elements 110 and/or the guide catheter energy delivery element 120.

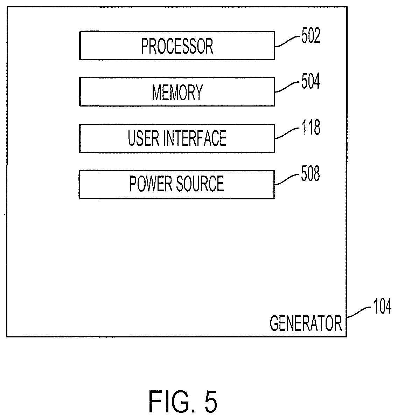

The generator 104 may have one or more processors 502, a memory 504, a user interface 118 and/or a power source 508, as shown in FIG. 5 for example. The one or more processors 502 may be electrically coupled to the memory 504, the user interface 118 and/or the power source 508. The one or more processors 502 may include one or more controllers that obtain an impedance signal that indicates the impedance at the treatment site or between or among energy delivery elements. The impedance may be related to the distance among the energy delivery elements and/or the positions or locations of the energy delivery elements.

Once the impedance is determined, the one or more processors 502 may determine the distance between and/or among the energy delivery elements and generate and render an image on the user interface 118 to show the location of the renal denervation device 102 within the blood vessel 304. The one or more processors 502 may control a state of each of the one or more energy delivery elements 110 and the amount of energy delivered to each of the one or more energy delivery elements 110 by the power source 508 and/or may provide an indication to the clinician. The one or more processors 502 may be coupled to the memory 504 and execute instructions that are stored in the memory 504.

The generator 104 may have a memory 504. The memory 504 may be coupled to the one or more processors 502 and store instructions that the one or more processors 502 executes. The memory 504 may include one or more of a Random Access Memory (RAM), Read Only Memory (ROM) or other volatile or non-volatile memory. The memory 504 may be a non-transitory memory or a data storage device, such as a hard disk drive, a solid-state disk drive, a hybrid disk drive, or other appropriate data storage, and may further store machine-readable instructions, which may be loaded and executed by the one or more processors 502.

The generator 104 may have a power source 508, such as a RF generator or other electrical source. The power source 508 provides a selected form and magnitude of energy for delivery to the treatment site via the renal denervation device 102. The generator 104 may have a user interface 118. The generator 104 may receive input, such as the selected form and the magnitude of energy to be delivered to each of the one or more energy delivery elements 110 and/or the guide catheter energy delivery element 120 and/or an indication to terminate or discontinue energy delivery, via the user interface 118.

The user interface 118 may include an input/output device that receives user input from a user interface element, a button, a dial, a microphone, a keyboard, or a touch screen. The user interface 118 may provide an output to an output device, such as a display, a speaker, an audio and/or visual indicator, or a refreshable braille display. The output may be an image of an overlay of the renal denervation device 102 within an image of the blood vessel 304. The output device may display an alert or notification or other information to the clinician and/or to confirm status and/or commands from the clinician. The output device may be an audio output device that outputs an audio indicator that indicates the notification or information to be provided to the clinician.

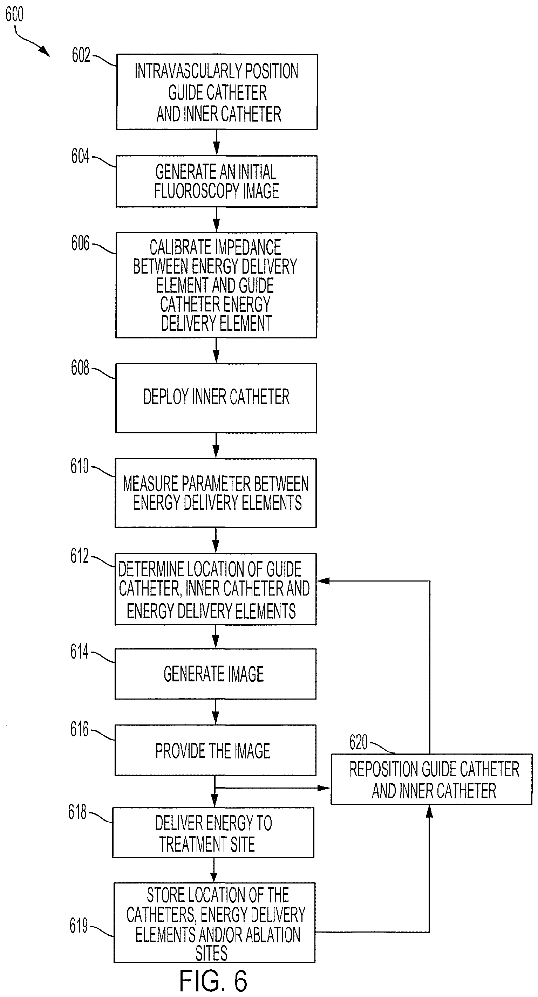

FIG. 6 is a flow diagram of a process 600 for providing an image of the renal denervation device within the blood vessel 304. One or more computers or one or more data processing apparatuses, for example, the processor 502 of generator 104 of the therapeutic assembly 100 of FIG. 1, appropriately programmed and/or using one or more other components, such as the one or more sensors 112, the guide catheter energy delivery element 120 and/or the one or more energy delivery elements 110, may implement the process 600.

The guide catheter 108 may be intravascularly delivered and/or positioned within a blood vessel 304, such as a vessel that is at or proximate to a renal artery of the human patient (602). The guide catheter 108 including the guide catheter energy element 120 may be delivered and/or positioned at or proximate to the blood vessel 304, such as the renal artery. The inner catheter 114 may be in a delivery configuration where the inner catheter 114 is entirely or substantially within the lumen of the guide catheter 108 when the guide catheter 108 is delivered and/or positioned at or proximate to the blood vessel 304.

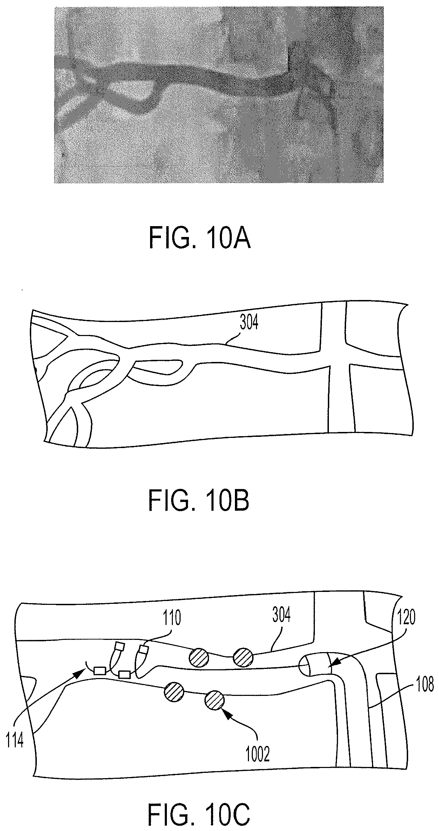

The therapeutic assembly 100 may obtain an initial fluoroscopy image (604). The clinician may inject or deliver a contrast dye into the blood vessel 304 to better visualize the structure of the blood vessel 304 and use a scanner, such as an X-ray scanner, to generate the initial fluoroscopy image. The initial fluoroscopy image may capture an image of the blood vessel 304, such as the artery, and as shown in FIG. 10A, for example, and/or capture an image of the renal denervation device 102 including an initial position of the guide catheter 108 and/or the inner catheter 114 where the distal ends of the guide catheter 108 and the inner catheter are substantially aligned. The initial fluoroscopy image may be taken from different vantage points and perspectives to capture the blood vessel 304, such as an interior and/or posterior view, to better determine the anatomy of the blood vessel 304. The initial fluoroscopy image may be provided to the generator 104, which may store the initial fluoroscopy image within the memory 504.

Once the renal denervation device 102 is positioned at or near the treatment site within the blood vessel 304 and before the inner catheter 114 is deployed from the guide catheter 108, the therapeutic assembly 100 may calibrate one or more parameters, such as the impedance of the signal (606). The guide catheter 108 may deploy an expandable element 122 to stabilize the guide catheter 108 and/or the guide catheter energy delivery element 120 relative to the energy delivery element 110 before and/or after calibration. The therapeutic assembly 100 may calibrate the impedance between the guide catheter energy delivery element 120 and one or more energy delivery elements 110, such as the first energy delivery element 110a or the second energy delivery element 110b. The generator 104 may deliver or output a signal, such as an impedance or an electrical signal, from the guide catheter energy delivery element 120 and that is detected by one of the one or more energy delivery elements 110, such as the distalmost energy delivery element on the inner catheter 114. Since the inner catheter 114 is in the delivery configuration where the inner catheter 114 is entirely within or substantially within the lumen of the guide catheter 108, this detected impedance corresponds to an initial undeployed position of the inner catheter 114 (which may hereinafter be referred to as an "initial impedance") where the inner catheter 114 has not extended away from the distal portion of the guide catheter 108 into the deployed configuration. The initial impedance is used to "zero-out" the impedance and determine the change in the impedance due to the change in location or position of the inner catheter 114 relative to the guide catheter 108. This initial impedance may represent a natural impedance between two energy delivery elements when the two energy delivery elements are substantially adjacent and/or aligned with each other, such as the guide catheter energy delivery element 120 and the first energy delivery element 110a when the inner catheter 114 is in the delivery configuration and substantially within the guide catheter 108.

Once the impedance is calibrated, the therapeutic assembly 100 may be deployed to perform the renal denervation (RDN) procedure (608). A guidewire 206 that extends out and away from the distal tip 202 of the inner catheter 114 may be advanced through the blood vessels to the target location. The inner catheter 114 is then advanced over the guidewire to the intended treatment location. The guidewire 206 is then retracted or withdrawn within the distal tip 202, proximal of the most proximal energy delivery elements which causes the shape of the inner catheter 114 to change from the low-profile configuration to the expanded deployed configuration. In the expanded deployed configuration, the one or more energy delivery elements 110 may be in apposition to the wall of the blood vessel 304 and be arranged in a generally helical or spiral configuration.

When the inner catheter 114 is in the deployed configuration, the therapeutic assembly 100 measures the one or more parameters, such as the impedance of a signal (610). The renal denervation device 102 may propagate a signal, such as an electrical signal or an impedance signal, between or among two or more energy delivery elements. For example, the renal denervation device 102 may propagate the signal between the guide catheter energy delivery element 120 and the one or more energy delivery elements 110, such as the distalmost or first energy delivery element 110a. The guide catheter energy delivery element 120 may emit the signal and the distalmost or first energy delivery element 110a may detect the signal. The therapeutic assembly 100 may measure the impedance between the two or more energy delivery elements and/or may measure the one or more parameters of the signal, such as the voltage, signal attenuation, propagation time and/or propagation signal, and determine the impedance from the one or more parameters of the signal.

The therapeutic assembly 100 determines the location of the guide catheter 108, the inner catheter 114 and/or the energy delivery elements 110, 120 (612). The therapeutic assembly 100 determines the location of the renal denervation device 102 including the location of the inner catheter 114 and/or the one or more energy delivery elements 110 based on the initial impedance and the measured impedance between energy delivery elements 110, 120. As the inner catheter 114 is advanced distally and extended away from the guide catheter 108 the impedance between the inner catheter 114 and the guide catheter 108 changes. In particular, as the inner catheter 114 is deployed and extends away from the guide catheter 108 into the deployed configuration, the impedance increases because there is more medium, such as blood or tissue, in between the one or more energy delivery elements 110 and the guide catheter energy delivery element 120. Moreover, as the inner catheter 114 is retracted and drawn toward the guide catheter back into the delivery configuration the impedance decreases because there is less medium in between the one or more energy delivery elements 110 and the guide catheter energy delivery element 120.

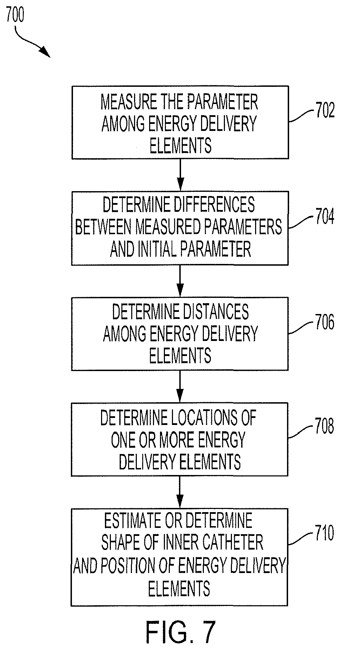

The therapeutic assembly 100 may compare the measured impedance, such as between the guide catheter energy delivery element 120 and the one or more energy delivery elements 110, with the initial impedance, which was calibrated when the inner catheter 114 was substantially within or entirely within the guide catheter 108. The change in the impedance may be related to and correspond to the relative distance between the one or more energy delivery elements 110 and the guide catheter energy delivery element 120. And thus, the change in the impedance may correspond to the relative distance that the inner catheter 114 has extended away from the guide catheter 108. For example, as the measured change in the impedance increases, the relative distance that the inner catheter 114 has extended away from the guide catheter 108 is greater. And, as the measured change in the impedance decreases, the relative distance that the inner catheter 114 has extended away from the guide catheter 108 is less. That is, the impedance may directly correspond and/or may be directly proportional to the relative distance between the inner catheter 114 and the guide catheter 108. FIG. 7 further describes the process 700 for determining the location of the renal denervation device 102 including the one or more energy delivery elements 110.

In some implementations, the therapeutic assembly 100 may also determine the locations of the ablation sites 1002. The locations of the ablations sites may be determined automatically via an algorithm based on where the locations of the energy delivery elements 110, 120 were located when energy was or is to be delivered and/or based on user input from the clinician that indicates the location of the ablation sites 1002.

The therapeutic assembly 100 generates an image of the renal denervation device 102 including the inner catheter 114 and/or the one or more energy delivery elements 110 within the blood vessel 304 (614). The therapeutic assembly 100 may generate the image of the renal denervation device 102 within the blood vessel 304 based on the initial fluoroscopy image and the location of the renal denervation device 102. The therapeutic assembly 100 may also include the guide catheter 108, the guide catheter energy element 120 and/or previous ablation sites in the image. The previous ablation sites and/or the initial fluoroscopy image may be obtained from the memory 504. The therapeutic assembly 100 may generate and overlay an outline of the renal denervation device 102 at the determined location on top of the initial fluoroscopy image, which shows the initial location of the renal denervation device 102. The image may be generated with different vantage points to allow for a view of the renal denervation device 102 from different perspectives. When the therapeutic assembly overlays the renal denervation device 102 on top of the initial fluoroscopy image, the therapeutic assembly 100 may overlay the location of the guide catheter 108 and the location of the inner catheter 114 including the one or more energy delivery elements 110 on top of the initial fluoroscopy image of the blood vessel 304. And thus, the generated image may show the current location of the renal denervation device 102 within the blood vessel 304 and/or may show the change or movement of the renal denervation device 102 within the blood vessel 304 from its initial position.

The image may include an overlay of the renal denervation device on top of and/or including the blood vessel 304. By overlaying the renal denervation device 102 on top of and/or including the blood vessel 304, the image shows an estimation of the position and/or location of the renal denervation device 102 including the position and/or location of the inner catheter 114 and/or energy delivery elements 110, 120 within the blood vessel 304. Moreover, the image may identify the one or more locations for each of the one or more energy delivery elements 110 and their corresponding treatment sites within the blood vessel 304. This provides an image that visualizes the location and/or position of the renal denervation device 102 within the blood vessel 304 so that the clinician may identify the one or more locations that are being treated during ablation.

In some implementations, the therapeutic assembly overlays the current location of the renal denervation device 102 on top of and/or including the initial location and previous locations of the renal denervation device 102 and/or ablations sites within the blood vessel 304. This provides an image that visualizes the movement of the location and/or position of the renal denervation device 102 within the blood vessel 304 as the inner catheter 114 is transformed into the deployed configuration.

The therapeutic assembly 100 provides or outputs the generated image (616). The therapeutic assembly 100 may provide or output the generated image on the user interface 118, such as on a display of the user interface 118, and as shown in FIGS. 10B and 10C, for example. The generated image may show portions of the guide catheter 108, the inner catheter 114, and/or energy delivery elements 110, 120 as an overlay on and/or within the blood vessel 304, such as the renal artery. The generated image may show the location of one or more ablation sites 1002 where previous ablations occurred. The locations of the ablation sites 1002 may be determined automatically via an algorithm based on where the locations of the energy delivery elements 110, 120 were located when energy was delivered and/or based on user input from the clinician that indicates the location of the ablation sites 1002. The locations of the guide catheter 108, the inner catheter 114, energy delivery elements 110, 120 and/or ablation sites 1002 may be plotted as an overlay over a previous image, such as the initial fluoroscopy image, to show or track the movement or positions of the guide catheter 108, the inner catheter 114, the energy delivery elements 110, 120 and/or the ablation sites 1002. In some implementations, only a portion of the guide catheter 108 and/or a portion of the inner catheter 114 where the energy delivery elements 110, 120 are positioned on the guide catheter 108 and/or the inner catheter 114 may be plotted. For example, other aspects of the guide catheter 108 and/or the inner catheter 114 may not be plotted because they are non-conductive (e.g., a polymer shaft) or electrically isolated, such as the radiopaque marker band 204, which may or may not have electrical connections.

This allows the clinician to visualize the position and/or location of the renal denervation device 102 within the blood vessel 304 without the need to inject or deliver contrast dye to perform fluoroscopy. And thus, using the impedance and the relationship between the impedance and the relative distance between two energy delivery elements 110, 120, the therapeutic assembly 100 reduces the amount of contrast needed for RDN procedure and reduces the amount of fluoroscopy imaging required, which would lessen the radiation exposure to the clinician and/or patient. Moreover, the plot may show previous ablation sites 1002, and this allows the clinician to visualize the position and/or location of the previous ablation sites 1002 to avoid ablating in a location where a previous ablation has occurred to avoid damaging tissue that has already been ablated. This allows the clinician to perform a series of ablations and to stagger the ablations to avoid overlap.

The clinician may reposition the guide catheter 108 and/or the inner catheter 114 should the clinician desire to ablate a different location than the current location provided or shown on the image (620). The repositioning of the guide catheter 108 and/or the inner catheter 114 is further described below. Otherwise, the therapeutic assembly 100 may deliver energy to the treatment site (618).

The therapeutic assembly may deliver energy via the one or more energy delivery elements 110 and/or the guide catheter energy delivery element 120. The generator 104 may provide the neuromodulation energy through the one or more energy delivery elements 110 and/or the guide catheter energy delivery element 120 to stimulate the nerves proximate to the treatment site (e.g., the wall of the blood vessel 304). For example, the generator 104 may provide radio frequency (RF) energy to ablate the nerves proximate to the treatment site via one or more energy delivery elements 110, such as one or more electrodes.

The therapeutic assembly 100 stores the location of the guide catheter 108, the inner catheter 114, the energy delivery elements 110, 120 and/or the ablation sites 1002 (619). The locations of the guide catheter 108, the inner catheter 114, the one or more energy delivery elements 110 and/or the guide catheter energy element 120 may be associated with a timestamp and/or a treatment plan that includes the type, amount and/or duration of the RDN therapy to be performed, and which may also be stored along with the locations of the components of the renal denervation device 102 in the memory 504. The therapeutic assembly 100 may store the locations for multiple treatments over a period of time, which allows the therapeutic assembly 100 to track the movement of the inner catheter 114, the guide catheter 108 and/or the energy delivery elements 110, 120 including the movement of the various components over the period of time. By capturing the locations of the energy delivery elements 110, 120, the therapeutic assembly 100 may provide or plot the locations of the ablation sites for a clinician on the user interface 118 in real-time including during the course of treatment. A treatment may be a session of medical care, which may involve a single instance of energy delivery or multiple deliveries of instances of energy delivery during a single visit. In some implementations, a treatment may refer to a single instance of energy delivery.

The clinician may reposition the guide catheter 108, the inner catheter 114 and/or one or more energy delivery elements 110 to perform ablation when the renal denervation therapy is not complete (620). The clinician may use the handle 116 to guide and/or advance a distal portion of the guide catheter 108 through the blood vessels of the patient, such as a human patient, to a target location of a blood vessel and remotely manipulate the distal portion of the guide catheter 108 and/or the inner catheter 114. The handle 116 may be used to manipulate the inner catheter 114 between the delivery configuration and the expanded deployed configuration. During repositioning, the therapeutic assembly 100 may re-determine the location of the guide catheter 108, the inner catheter 114 and/or the energy delivery element 110, 120 to display and plot on the image (612). This allows the clinician to track the movement of the guide catheter 108, the inner catheter 114 and/or the energy delivery elements 110, 120 along with the ablation sites 1002 in real-time.

FIG. 7 is a flow diagram of a process 700 for determining the locations of one or more energy delivery elements 110. One or more computers or one or more data processing apparatuses, for example, the processor 502 of generator 104 of the therapeutic assembly 100 of FIG. 1, appropriately programmed and/or using one or more other components, such as the one or more sensors 112, the guide catheter energy delivery element 120 and/or the one or more energy delivery elements 110, may implement the process 700.

Once the therapeutic assembly 100 has calibrated the parameter, such as the impedance, among the energy delivery elements 110, 120 and the inner catheter 114 has been deployed, the therapeutic assembly 100 measures the parameter, such as the impedances, among the guide catheter energy delivery element 120 and/or the one or more energy delivery elements 110 (702). For example, the therapeutic assembly 100 may measure a first impedance between the guide catheter energy delivery element 120 and a first energy delivery element 110a and/or a second impedance between the guide catheter energy delivery element 120 and a second energy delivery element 110b. The therapeutic assembly 100 may perform any number of measurements of the impedance between the guide catheter energy delivery element 120 and a corresponding energy delivery element 110 or between different energy delivery elements 110. Since the therapeutic assembly 100 may perform measurements of the impedance between different combinations among the one or more energy delivery elements and/or the guide catheter energy delivery element 120 and the measured impedances correlate with the relative distances among the different energy delivery elements, the therapeutic assembly 100 may determine relative locations of the various energy delivery elements among each other.

Given the measured impedance among the various one or more energy delivery elements 110 and/or the guide catheter energy delivery element 120, the therapeutic assembly 100 may determine or calculate the difference in the measured impedance and the calibrated or initial impedance (704). The therapeutic assembly 100 determines the difference between the measured impedance of two or more energy delivery elements and the initial impedance, which may reflect the natural impedance between two or more energy delivery elements that are adjacent and/or aligned with each other. The difference between the measured impedance and the initial impedance may be reflective of or associated with the distance between the two or more energy delivery elements, the amount of surface area of the energy delivery element is in contact with the wall of the blood vessel 304 and/or the amount of surface area of the energy delivery element is exposed to the medium flowing through the blood vessel 304.

The therapeutic assembly 100 may determine one or more distances among the one or more energy delivery elements 110 and/or the guide catheter energy delivery element 120 (706). The one or more distances may be relative distances among the one or more energy delivery elements 110 and/or the guide catheter energy delivery element 120. The therapeutic assembly 100 may determine or calculate the distance between two energy delivery elements based on the corresponding impedance measured between the two energy delivery elements, e.g., between the guide catheter energy delivery element 120 and the first energy delivery element 110a, the guide catheter energy delivery element 120 and the second energy delivery element 110b, the guide catheter energy delivery element 120 and the third energy delivery element 110c or the guide catheter energy delivery element 120 and the fourth energy delivery element 110d. In some implementations, the therapeutic assembly 100 may determine or calculate the distance between two energy delivery elements 110 positioned on the inner catheter 114 based on the corresponding impedance between the two energy delivery elements 110. For example, the therapeutic assembly 100 may determine the distance between the first energy delivery element 110a and the second energy delivery element 110b based on the impedance measured between the first energy delivery element 110a and the second energy delivery element 110b. The impedance may be directly proportional or correlated with the distance between the energy delivery elements 110, 120. For example, as the impedance increases, this may be representative of a greater or an increased relative distance between the two energy delivery elements, and as the impedance decreases, this may be representative of a lesser or a decreased relative distance between the two energy delivery elements.

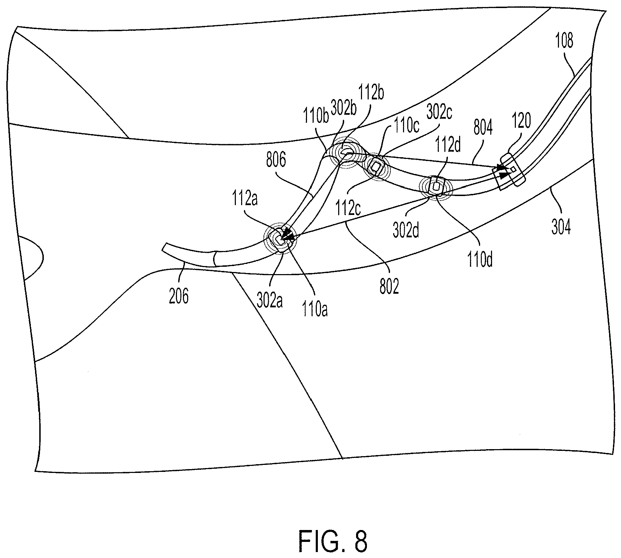

Once the relative distances among the one or more energy delivery element 110 and the guide catheter energy delivery element 120 are determined, the therapeutic assembly 100 may determine the locations of the one or more energy delivery elements 110 (708). The therapeutic assembly 100 may determine the locations of the one or more energy delivery elements 110 based on the one or more distances. For example, the therapeutic assembly 100 may determine a first relative distance 802 based on the impedance between the guide catheter energy delivery element 120 and the first energy delivery element 110a, a second relative distance 804 between the guide catheter energy delivery element 120 and the second energy delivery element 110b and a third relative distance 806 between the first energy delivery element 110a and the second energy delivery element 110b. Once the first relative distance 802, the second relative distance 804 and the third relative distance 806 are determined, the therapeutic assembly 100 may determine the locations of the first energy delivery element 110a, the second energy delivery element 110b and the guide catheter energy delivery element 120 based on the relative distances among the three energy delivery elements. Since the first energy delivery element 110a is further distal than the second energy delivery element 110b and the energy delivery elements 110 are positioned on the inner catheter 114 that is deployed away from the guide catheter 108, the therapeutic assembly may perform triangulation and/or trilateration of the three energy delivery elements and their relative distances to determine the locations of the three energy delivery elements and the compression or expansion of the helical shape of the deployed configuration and the amount of surface area each energy delivery element is in contact with the wall of the blood vessel 304, as shown in FIG. 8 for example. Similarly, the triangulation and/or trilateration may be performed on any combination of three energy delivery elements to determine the locations of the three energy delivery elements.

Once the location of each of the one or more energy delivery elements 110, the location of the inner catheter 114 and the location of the guide catheter 108 are determined or otherwise known within the blood vessel 304, the therapeutic assembly 100 may estimate or determine the shape of the inner catheter 114 when the inner catheter 114 is in the deployed configuration (710). For example, the therapeutic assembly 100 may estimate the amount and direction that the inner catheter 114 is flexed, bent or otherwise angled between energy delivery elements 110, 120 when the inner catheter 114 is in the deployed configuration based on the locations of the energy delivery elements 110, 120. The therapeutic assembly 100 may estimate where each of the one or more energy delivery elements 110 contact and/or are substantially near a wall of the blood vessel 304. This allows the therapeutic assembly 100 to more accurately generate the image of the inner catheter 114 and/or guide catheter 108 within the blood vessel 304 and render the position of the inner catheter 114 and the locations of the one or more energy delivery elements 110, as described above in FIG. 6.

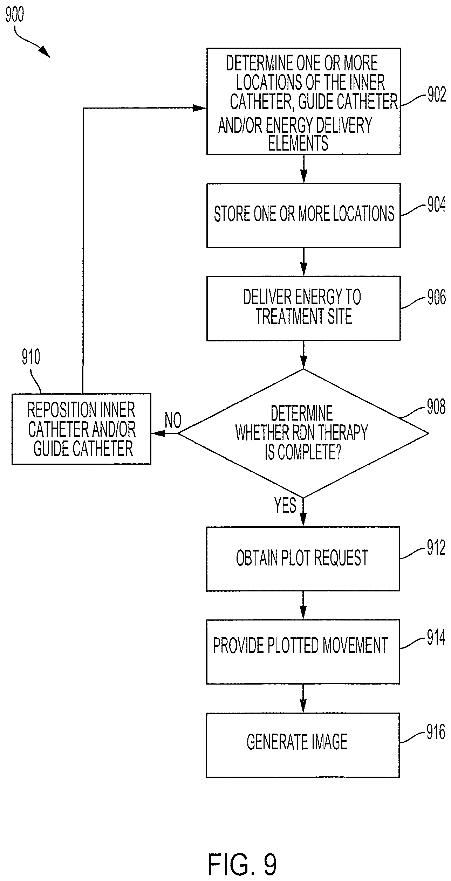

FIG. 9 is a flow diagram of a process 900 for tracking and plotting the movement of the inner catheter 114, the energy delivery elements 110, 120 and/or the guide catheter 108 within the blood vessel 304 to form a treatment plan or an archive of treatments. One or more computers or one or more data processing apparatuses, for example, the processor 502 of generator 104 of the therapeutic assembly 100 of FIG. 1, appropriately programmed and/or using one or more other components, such as the one or more sensors 112, the guide catheter energy delivery element 120 and/or the one or more energy delivery elements 110, may implement the process 900.

The therapeutic assembly 100 may determine the location of the inner catheter 114, the guide catheter 108 and/or the energy delivery elements 110, 120, as described above (902). The therapeutic assembly 100 may store the locations of the inner catheter 114, the guide catheter 108, the one or more ablation sites 1002 and/or the energy delivery elements 110, 120, as described above (904). The locations of the guide catheter 108, the inner catheter 114, the one or more energy delivery elements 110, the one or more ablation sites 1002 and/or the guide catheter energy element 120 may be associated with a timestamp and/or a treatment plan that includes the type, amount and/or duration of the RDN therapy to be performed, and which may also be stored along with the locations in the memory 504. The therapeutic assembly 100 may store the locations for multiple treatments over a period of time, which allows the therapeutic assembly 100 to track the movement of the inner catheter 114, the guide catheter 108 and/or the energy delivery elements 110, 120 including the movement of the various components over the period of time. The storing of the locations is a recording of the locations that may be archived and later accessed to allow for reference to it by the clinician. By capturing the locations of the energy delivery elements 110, 120, the therapeutic assembly 100 may provide the locations of the ablation sites 1002 along with the locations of the inner catheter 114, the guide catheter 108 and/or energy delivery elements 110, 120 to an operator or clinician on the user interface 118.

The therapeutic assembly 100 delivers energy to the treatment site within the blood vessel (906). The generator 104 may provide the neuromodulation energy through the one or more energy delivery elements 110 and/or the guide catheter energy delivery element 120 to stimulate the nerves proximate to the treatment site (e.g., the wall of the blood vessel 304). For example, the generator 104 may provide radio frequency (RF) energy to ablate the nerves proximate to the treatment site via one or more energy delivery elements 110, such as one or more electrodes. In some implementations, the therapeutic assembly 100 may provide pulsed electrical energy, microwave energy, optical energy, ultrasound energy (e.g., intravascularly delivered ultrasound and/or high-frequency ultrasound), direct heat energy, radiation, cryogenic cooling, chemical-based treatment, and/or another suitable type of neuromodulation energy. The determination of the one or more locations of the guide catheter 108, the inner catheter 114, and the one or more energy delivery elements and the delivery of energy is further described above in FIG. 6.

The therapeutic assembly 100 may determine whether the RDN therapy is complete (908). The therapeutic assembly 100 may measure and compare a parameter, such as a morning surge blood pressure or a nocturnal blood pressure, that is indicative of a risk factor associated hypertension before and after the delivery of energy to the treatment site and determine whether RDN therapy was successful based on the comparison. For example, if there is an improvement of approximately 5%, 10% or at least 20% over a period of time of approximately of a few minutes, a half hour, an hour, a few days, a few weeks or more, the therapeutic assembly 100 may determine that RDN therapy is complete. In some implementations, the parameter may be the impedance between an energy delivery element 110 and a return electrode, such as a ground patch. For example, if there is a reduction in impedance of approximately 5%, 10% or at least 20% over a period of time of approximately of a few minutes or over a few hours, this may signify that tissue ablation has occurred. In some implementations, the therapeutic assembly 100 may receive user input, such as from the clinician, that indicates whether RDN therapy is complete.

When the therapeutic assembly 100 determines that RDN therapy is not complete, the clinician may reposition the renal denervation device 102 including the inner catheter 114 and/or the guide catheter 108 within the blood vessel 304 to a second treatment location (910). Once repositioned, the therapeutic assembly 100 may re-determine the locations of the various components (902) and repeat the RDN therapy. Otherwise, when the therapeutic assembly 100 determines that RDN therapy is complete, the therapeutic assembly 100 may provide information regarding the one or more locations where ablation was performed to the clinician.

The therapeutic assembly 100 may obtain a plot request (912). The plot request may be received via user input on the user interface 118 and may be a request to plot, map or otherwise provide or output the locations of the guide catheter 108, the inner catheter 114, the one or more energy delivery elements 110, 120 and/or the ablation sites 1002 over the period of time, over multiple treatments and/or over multiple treatment plans. For example, the request may be to map the locations of the renal denervation device 102, the ablation sites 1002 and/or the guide catheter 108 over multiple treatments, e.g., multiple sessions of treatments over a period of weeks or months so that the clinician ensures that ablation is performed at different sites and not at the same ablation sites during the course of the multiple treatments. This allows the clinician to change the treatment sites when ablation is not effective or only effective so that the clinician may perform ablation at treatments sites that would be more effective in subsequent treatments. The therapeutic assembly 100 may obtain the one or more locations of the renal denervation device 102, the guide catheter 108 and/or the ablation sites 1002 over the period of time, over multiple treatments and/or over multiple treatment plans from the memory 504.

The therapeutic assembly 100 may provide the plotted movement (914). The therapeutic assembly 100 may provide a log of the plotted movement of the inner catheter 114, the guide catheter 108, the energy delivery elements 110, 120 and/or the ablation sites 1002. In some implementations, the therapeutic assembly 100 may generate an image that shows the plotted movement of the inner catheter 114 and the energy delivery elements 110, 120 so that the clinician is not repeating the ablation in the same location and output the image to the display. The image may be a combination of images that are overlaid on each other over the period of time, over the multiple treatments and/or or over the multiple treatment plans based on the one or more locations of the renal denervation device 102. The image may show the movement of the locations including any change in location of the inner catheter 114, the locations of the one or more energy delivery element 110, the location of the ablation sites 1002 and/or the location of the guide catheter 108 within the blood vessel 304 over the period of time, multiple treatments and/or multiple treatment plans.

The therapeutic assembly 100 renders the generated image (916). The therapeutic assembly 100 may render the generated image on the user interface 118, such as on a display, and as described above in FIG. 6 for example. The image may include other additional information. For example, the memory 504 may have stored the other additional information along with the corresponding location of the renal denervation device 102. The other additional information may include the type, amount, frequency or other parameter, such as the affect, of the performed ablation. The other additional information may be provided or shown to the clinician when the generated image is rendered so that clinician may determine the complete history of the performed ablation during the period of time, treatment and/or treatment plan.

Exemplary embodiments of the invention have been disclosed in an illustrative style. Accordingly, the terminology employed throughout should be read in a non-limiting manner. The invention is defined by the claims.

## Claims

1. A system (100) for renal denervation, comprising:
a guide catheter (108) having a lumen and a guide catheter energy delivery element (120) positioned at a distal portion of the guide catheter (108), the distal portion of the guide catheter (108) being configured to be intravascularly positioned within a main vessel;
a renal denervation device (102) comprising an inner catheter (114) positioned within the lumen of the guide catheter (108) and having a plurality of energy delivery elements (110) including a first energy delivery element (110a) and a second energy delivery element (110b), a distal portion of the inner catheter (114) being configured to be positioned near a treatment site within a renal blood vessel and transform from a delivery configuration to a deployed configuration; and
a controller coupled to the renal denervation device (102) and configured to:
measure a first impedance between the first energy delivery element (110a) and the guide catheter energy delivery element (120), and
provide a location of the inner catheter (114) within the renal blood vessel based on the first impedance.

2. The system (100) of claim 1, wherein when the inner catheter (114) is in the delivery configuration the inner catheter (114) is substantially within the lumen of the guide catheter (108) and the first energy delivery element (110a) is aligned with the guide catheter energy delivery element (120).

3. The system (100) of claim 2, wherein the controller is configured to:
calibrate the impedance between the first energy delivery element (110a) and the guide catheter energy delivery element (120) when the inner catheter (114) is in the delivery configuration.

4. The system (100) of any of the preceding claims, wherein when the inner catheter (114) is in the deployed configuration the inner catheter (114) is extended away from the guide catheter a(108) nd a distal portion of the inner catheter (114) expands into a helical or spiral configuration to place the energy delivery element (110) at or near the treatment site.

5. The system (100) of claim 4, wherein the controller is configured to:
measure the first impedance between the first energy delivery element (110a) and the guide catheter energy delivery element (120) when the inner catheter (114) is in the delivery configuration;
measure a second impedance between the first energy delivery element (110a) and the guide catheter energy delivery element (120) when the inner catheter (114) is in the deployed configuration;
determine a difference between the second impedance and the first impedance; and
determine the location of the inner catheter (114) based on the difference.

6. The system (100) of claim 4, wherein the controller is configured to:
measure the first impedance between the first energy delivery element (110a) and the guide catheter energy delivery element (120) when the inner catheter (114) is in the delivery configuration;
measure a second impedance between the first energy delivery element (110a) and the guide catheter energy delivery element (120) when the inner catheter (114) is in the deployed configuration; and
measure a third impedance between the second energy delivery element (110b) and the guide catheter energy delivery element (120) when the inner catheter (114) is in the deployed configuration.

7. The system (100) of claim 6, wherein the controller is configured to:
determine a first difference between the second impedance and the first impedance;
determine a second difference between the third impedance and the first impedance; and
determine the location of the inner catheter (114) including a location of the first energy delivery element (110a) and a location of the second delivery element (110b) based on the first difference and the second difference.

8. The system (100) of any of the preceding claims, further comprising:
a display configured to render an image of the renal blood vessel, the guide catheter (108) and the inner catheter (114) including the first energy delivery element (110a) and the second energy delivery element (110b) within the renal blood vessel; or further comprising:
a display configured to render images, wherein the controller is configured to:
generate an image of the renal blood vessel, the guide catheter (108) and the inner catheter (114) within the renal blood vessel, and
cause the image to be rendered on the display.

9. The system (100) of claim 1,
wherein the distal portion of the guide catheter (108) is configured to be intravascularly positioned within a renal blood vessel;
wherein the inner catheter (114) is configured to be positioned near a treatment site within the renal blood vessel; and the system (100) further comprising:
a display configured to render images;
wherein the controller is coupled to the guide catheter (108), the inner catheter (114) and the display and configured to:
render, on the display, an image of the guide catheter (108) and the inner catheter (114) based on the location of inner catheter (114) within the renal blood vessel.

10. The system (100) of claim 9, wherein the guide catheter energy delivery element (120) is positioned on a distal portion of the guide catheter, and wherein the energy delivery element (110) is positioned on the inner catheter (114).

11. The system (100) of claim 9 or 10, wherein the inner catheter (114) is configured to transform from a delivery configuration to a deployed configuration.

12. The system (100) of claim 11, wherein the controller is configured to:
measure the first impedance between the energy delivery element (110) and the guide catheter energy delivery element (120) when the inner catheter (114) is in the delivery configuration;
measure a second impedance between the first energy delivery element (110) and the guide catheter energy delivery element (120) when the inner catheter (114) is in the deployed configuration;
determine a difference between the second impedance and the first impedance; and
determine the location of the inner catheter (114) based on the difference.

13. The system (100) of any of claims 9-12, further comprising:
a generator electrically coupled to the guide catheter electrode and the energy delivery element (110), and configured to deliver neuromodulation energy to the treatment site within the renal blood vessel.

14. The system (100) of any of claims 9-11 and 13, wherein the controller is configured to:
measure a second impedance between the energy delivery element (110) and the guide catheter energy delivery element (120);
determine a second location of the inner catheter device (114) based on the second impedance; and
render, on the display, a second image of a second location of the inner catheter (114) within the renal blood vessel.

15. The system (100) of claim 14, further comprising:
a memory configured to store the first location and the second location of the inner catheter (114);
wherein the controller is configured to:
render, on the display, a third image that includes a plot of the inner catheter (114) from the first location to the second location.

## Patentansprüche

1. System (100) für Nierendenervation, umfassend:
einen Führungskatheter (108) mit einem Lumen und einem Führungskatheterenergieabgabeelement (120), das an einem distalen Abschnitt des Führungskatheters (108) positioniert ist, wobei der distale Abschnitt des Führungskatheters (108) dazu ausgelegt ist, intravaskulär in einem Hauptgefäß positioniert zu sein;
eine Nierendenervationsvorrichtung (102), die einen Innenkatheter (114) umfasst, der innerhalb des Lumens des Führungskatheters (108) positioniert ist und mehrere Energieabgabeelemente (110) aufweist, die ein erstes Energieabgabeelement (110a) und ein zweites Energieabgabeelement (110b) umfassen, wobei ein distaler Abschnitt des Innenkatheters (114) dazu ausgelegt ist, nahe einer Behandlungsstelle innerhalb eines Nierenblutgefäßes positioniert zu werden und von einer Zuführauslegung in eine entfaltete Auslegung überführt zu werden; und
eine Steuerung, die mit der Nierendenervationsvorrichtung (102) gekoppelt ist und ausgelegt ist zum:
Messen einer ersten Impedanz zwischen dem ersten Energieabgabeelement (110a) und dem Führungskatheterenergieabgabeelement (120), und
Bereitstellen einer Position des Innenkatheters (114) innerhalb des Nierenblutgefäßes basierend auf der ersten Impedanz.

2. System (100) nach Anspruch 1, wobei, wenn sich der Innenkatheter (114) in der Zuführauslegung befindet, der Innenkatheter (114) im Wesentlichen innerhalb des Lumens des Führungskatheters (108) ist und das erste Energieabgabeelement (110a) mit dem Führungskatheterenergieabgabeelement (120) ausgerichtet ist.

3. System (100) nach Anspruch 2, wobei die Steuerung ausgelegt ist zum:
Kalibrieren der Impedanz zwischen dem ersten Energieabgabeelement (110a) und dem Führungskatheterenergieabgabeelement (120), wenn sich der Innenkatheter (114) in der Zuführauslegung befindet.

4. System (100) nach einem der vorhergehenden Ansprüche, wobei, wenn sich der Innenkatheter (114) in der entfalteten Auslegung befindet, der Innenkatheter (114) von dem Führungskatheter (108) weg ausgefahren wird und sich ein distaler Abschnitt des Innenkatheters (114) in eine wendelförmige oder spiralförmige Auslegung ausdehnt, um das Energieabgabeelement (110) an oder in der Nähe der Behandlungsstelle zu platzieren.

5. System (100) nach Anspruch 4, wobei die Steuerung ausgelegt ist zum:
Messen der ersten Impedanz zwischen dem ersten Energieabgabeelement (110a) und dem Führungskatheterenergieabgabeelement (120), wenn sich der Innenkatheter (114) in der Zuführauslegung befindet;
Messen einer zweiten Impedanz zwischen dem ersten Energieabgabeelement (110a) und dem Führungskatheterenergieabgabeelement (120), wenn sich der Innenkatheter (114) in der entfalteten Auslegung befindet;
Bestimmen einer Differenz zwischen der zweiten Impedanz und der ersten Impedanz; und
Bestimmen der Position des Innenkatheters (114) basierend auf der Differenz.

6. System (100) nach Anspruch 4, wobei die Steuerung ausgelegt ist zum:
Messen der ersten Impedanz zwischen dem ersten Energieabgabeelement (110a) und dem Führungskatheterenergieabgabeelement (120), wenn sich der Innenkatheter (114) in der Zuführauslegung befindet;
Messen einer zweiten Impedanz zwischen dem ersten Energieabgabeelement (110a) und dem Führungskatheterenergieabgabeelement (120), wenn sich der Innenkatheter (114) in der entfalteten Auslegung befindet; und
Messen einer dritten Impedanz zwischen dem zweiten Energieabgabeelement (110b) und dem Führungskatheterenergieabgabeelement (120), wenn sich der Innenkatheter (114) in der entfalteten Auslegung befindet.

7. System (100) nach Anspruch 6, wobei die Steuerung ausgelegt ist zum:
Bestimmen einer ersten Differenz zwischen der zweiten Impedanz und der ersten Impedanz;
Bestimmen einer zweiten Differenz zwischen der dritten Impedanz und der ersten Impedanz; und
Bestimmen der Position des Innenkatheters (114) einschließlich einer Position des ersten Energieabgabeelements (110a) und einer Position des zweiten Abgabeelements (110b) basierend auf der ersten Differenz und der zweiten Differenz.

8. System (100) nach einem der vorhergehenden Ansprüche, das ferner Folgendes umfasst:
eine Anzeige, die dazu ausgelegt ist, ein Bild des Nierenblutgefäßes, des Führungskatheters (108) und des Innenkatheters (114) einschließlich des ersten Energieabgabeelements (110a) und des zweiten Energieabgabeelements (110b) innerhalb des Nierenblutgefäßes darzustellen; oder ferner umfassend:
eine Anzeige, die zum Darstellen von Bildern ausgelegt ist, wobei die Steuerung ausgelegt ist zum:
Erzeugen eines Bildes des Nierenblutgefäßes, des Führungskatheters (108) und des Innenkatheters (114) innerhalb des Nierenblutgefäßes, und
Veranlassen, dass das Bild auf der Anzeige dargestellt wird.

9. System (100) nach Anspruch 1,
wobei der distale Abschnitt des Führungskatheters (108) dazu ausgelegt ist, intravaskulär innerhalb eines Nierenblutgefäßes positioniert zu sein;
wobei der Innenkatheter (114) dazu ausgelegt ist, nahe einer Behandlungsstelle innerhalb des Nierenblutgefäßes positioniert zu werden; und wobei das System (100) ferner Folgendes umfasst:
eine Anzeige, die zum Darstellen von Bildern ausgelegt ist;
wobei die Steuerung mit dem Führungskatheter (108), dem Innenkatheter (114) und der Anzeige gekoppelt und ausgelegt ist zum:
Darstellen, auf der Anzeige, eines Bildes des Führungskatheters (108) und des Innenkatheters (114) basierend auf der Position des Innenkatheters (114) innerhalb des Nierenblutgefäßes.

10. System (100) nach Anspruch 9, wobei das Führungskatheterenergieabgabeelement (120) an einem distalen Abschnitt des Führungskatheters positioniert ist und wobei das Energieabgabeelement (110) an dem Innenkatheter (114) positioniert ist.

11. System (100) nach Anspruch 9 oder 10, wobei der Innenkatheter (114) dazu ausgelegt ist, von einer Zuführauslegung in eine entfaltete Auslegung überführt zu werden.

12. System (100) nach Anspruch 11, wobei die Steuerung ausgelegt ist zum:
Messen der ersten Impedanz zwischen dem Energieabgabeelement (110) und dem Führungskatheterenergieabgabeelement (120), wenn sich der Innenkatheter (114) in der Zuführauslegung befindet;
Messen einer zweiten Impedanz zwischen dem ersten Energieabgabeelement (110) und dem Führungskatheterenergieabgabeelement (120), wenn sich der Innenkatheter (114) in der entfalteten Auslegung befindet;
Bestimmen einer Differenz zwischen der zweiten Impedanz und der ersten Impedanz; und
Bestimmen der Position des Innenkatheters (114) basierend auf der Differenz.

13. System (100) nach einem der Ansprüche 9-12, das ferner Folgendes umfasst:
einen Generator, der elektrisch mit der Führungskatheterelektrode und dem Energieabgabeelement (110) gekoppelt ist und dazu ausgelegt ist, Neuromodulationsenergie an die Behandlungsstelle innerhalb des Nierenblutgefäßes abzugeben.

14. System (100) nach einem der Ansprüche 9-11 und 13, wobei die Steuerung ausgelegt ist zum:
Messen einer zweiten Impedanz zwischen dem Energieabgabeelement (110) und dem Führungskatheterenergieabgabeelement (120);
Bestimmen einer zweiten Position der Innenkathetervorrichtung (114) basierend auf der zweiten Impedanz; und
Darstellen, auf der Anzeige, eines zweiten Bildes einer zweiten Position des Innenkatheters (114) innerhalb des Nierenblutgefäßes.

15. System (100) nach Anspruch 14, das ferner Folgendes umfasst:
einen Speicher, der ausgelegt ist zum Speichern der ersten Position und der zweiten Position des Innenkatheters (114);
wobei die Steuerung ausgelegt ist zum:
Darstellen, auf der Anzeige, eines dritten Bildes, das einen Plot des Innenkatheters (114) von der ersten Position zu der zweiten Position aufweist.

## Revendications

1. Système (100) pour dénervation rénale, comprenant :
un cathéter guide (108) ayant une lumière et un élément de distribution d'énergie de cathéter guide (120) positionné au niveau d'une partie distale du cathéter guide (108), la partie distale du cathéter guide (108) étant configurée pour être positionnée de façon intravasculaire à l'intérieur d'un vaisseau principal ;
un dispositif de dénervation rénale (102) comprenant un cathéter interne (114) positionné à l'intérieur de la lumière du cathéter guide (108) et ayant une pluralité d'éléments de distribution d'énergie (110) incluant un premier élément de distribution d'énergie (110a) et un second élément de distribution d'énergie (110b), une partie distale du cathéter interne (114) étant configurée pour être positionnée près d'un site de traitement à l'intérieur d'un vaisseau sanguin rénal et se transformer d'une configuration de mise en place à une configuration déployée ; et
une unité de commande couplée au dispositif de dénervation rénale (102) et configurée pour :
mesurer une première impédance entre le premier élément de distribution d'énergie (110a) et l'élément de distribution d'énergie de cathéter guide (120), et
fournir un emplacement du cathéter interne (114) à l'intérieur du vaisseau sanguin rénal sur la base de la première impédance.

2. Système (100) de la revendication 1, dans lequel, lorsque le cathéter interne (114) est dans la configuration de mise en place, le cathéter interne (114) est sensiblement à l'intérieur de la lumière du cathéter guide (108) et le premier élément de distribution d'énergie (110a) est aligné avec l'élément de distribution d'énergie de cathéter guide (120).

3. Système (100) de la revendication 2, dans lequel l'unité de commande est configurée pour :
calibrer l'impédance entre le premier élément de distribution d'énergie (110a) et l'élément de distribution d'énergie de cathéter guide (120) lorsque le cathéter interne (114) est dans la configuration de mise en place.

4. Système (100) de quelconques des revendications précédentes, dans lequel lorsque le cathéter interne (114) est dans la configuration déployée le cathéter interne (114) est étendu en éloignement du cathéter guide (108) et une partie distale du cathéter interne (114) s'agrandit en une configuration hélicoïdale ou en spirale pour placer l'élément de distribution d'énergie (110) au niveau ou près du site de traitement.

5. Système (100) de la revendication 4, dans lequel l'unité de commande est configurée pour :
mesurer la première impédance entre le premier élément de distribution d'énergie (110a) et l'élément de distribution d'énergie de cathéter guide (120) lorsque le cathéter interne (114) est dans la configuration de mise en place ;
mesurer une deuxième impédance entre le premier élément de distribution d'énergie (110a) et l'élément de distribution d'énergie de cathéter guide (120) lorsque le cathéter interne (114) est dans la configuration déployée ;
déterminer une différence entre la deuxième impédance et la première impédance ; et
déterminer l'emplacement du cathéter interne (114) sur la base de la différence.

6. Système (100) de la revendication 4, dans lequel l'unité de commande est configurée pour :
mesurer la première impédance entre le premier élément de distribution d'énergie (110a) et l'élément de distribution d'énergie de cathéter guide (120) lorsque le cathéter interne (114) est dans la configuration de mise en place ;
mesurer une deuxième impédance entre le premier élément de distribution d'énergie (110a) et l'élément de distribution d'énergie de cathéter guide (120) lorsque le cathéter interne (114) est dans la configuration déployée ; et
mesurer une troisième impédance entre le second élément de distribution d'énergie (110b) et l'élément de distribution d'énergie de cathéter guide (120) lorsque le cathéter interne (114) est dans la configuration déployée.

7. Système (100) de la revendication 6, dans lequel l'unité de commande est configurée pour :
déterminer une première différence entre la deuxième impédance et la première impédance ;
déterminer une seconde différence entre la troisième impédance et la première impédance ; et
déterminer l'emplacement du cathéter interne (114) incluant un emplacement du premier élément de distribution d'énergie (110a) et un emplacement du second élément de distribution (110b) sur la base de la première différence et de la seconde différence.

8. Système (100) de quelconques des revendications précédentes, comprenant en outre :
une unité d'affichage configurée pour effectuer le rendu d'une image du vaisseau sanguin rénal, le cathéter guide (108) et le cathéter interne (114) incluant le premier élément de distribution d'énergie (110a) et le second élément de distribution d'énergie (110b) à l'intérieur du vaisseau sanguin rénal ; ou comprenant en outre :
une unité d'affichage configurée pour effectuer le rendu d'images, dans lequel l'unité de commande est configurée pour :
générer une image du vaisseau sanguin rénal, du cathéter guide (108), et du cathéter interne (114) à l'intérieur du vaisseau sanguin rénal, et
amener l'image à être rendue sur l'unité d'affichage.

9. Système (100) de la revendication 1,
dans lequel la partie distale du cathéter guide (108) est configurée pour être positionné de façon intravasculaire à l'intérieur d'un vaisseau sanguin rénal ;
dans lequel le cathéter interne (114) est configuré pour être positionné près d'un site de traitement à l'intérieur du vaisseau sanguin rénal ; et le système (100) comprenant en outre :
une unité d'affichage configurée pour effectuer le rendu d'images ;
dans lequel l'unité de commande est couplée au cathéter guide (108), au cathéter interne (114), et à l'unité d'affichage et configurée pour :
effectuer le rendu, sur l'unité d'affichage, d'une image du cathéter guide (108) et du cathéter interne (114) sur la base de l'emplacement de cathéter interne (114) à l'intérieur du vaisseau sanguin rénal.

10. Système (100) de la revendication 9, dans lequel l'élément de distribution d'énergie de cathéter guide (120) est positionné sur une partie distale du cathéter guide, et dans lequel l'élément de distribution d'énergie (110) est positionné sur le cathéter interne (114).

11. Système (100) de la revendication 9 ou 10, dans lequel le cathéter interne (114) est configuré pour se transformer d'une configuration de mise en place à une configuration déployée.

12. Système (100) de la revendication 11, dans lequel l'unité de commande est configurée pour :
mesurer la première impédance entre l'élément de distribution d'énergie (110) et l'élément de distribution d'énergie de cathéter guide (120) lorsque le cathéter interne (114) est dans la configuration de mise en place ;
mesurer une deuxième impédance entre le premier élément de distribution d'énergie (110) et l'élément de distribution d'énergie de cathéter guide (120) lorsque le cathéter interne (114) est dans la configuration déployée ;
déterminer une différence entre la deuxième impédance et la première impédance ; et
déterminer l'emplacement du cathéter interne (114) sur la base de la différence.

13. Système (100) de quelconques des revendications 9 à 12, comprenant en outre :
un générateur couplé électriquement à l'électrode de cathéter guide et à l'élément de distribution d'énergie (110), et configuré pour distribuer de l'énergie de neuromodulation au site de traitement à l'intérieur du vaisseau sanguin rénal.

14. Système (100) de quelconques des revendications 9 à 11, et 13, dans lequel l'unité de commande est configurée pour :
mesurer une deuxième impédance entre l'élément de distribution d'énergie (110) et l'élément de distribution d'énergie de cathéter guide (120) ;
déterminer un second emplacement du cathéter interne dispositif (114) sur la base de la deuxième impédance ; et
effectuer le rendu, sur l'unité d'affichage, d'une deuxième image d'un second emplacement du cathéter interne (114) à l'intérieur du vaisseau sanguin rénal.

15. Système (100) de la revendication 14, comprenant en outre :
une mémoire configurée pour stocker le premier emplacement et le second emplacement du cathéter interne (114) ;
dans lequel l'unité de commande est configurée pour :
effectuer le rendu, sur l'unité d'affichage, d'une troisième image qui inclut un tracé du cathéter interne (114), du premier emplacement au second emplacement.
